# EUROPEAN PATENT SPECIFICATION

(11) **EP 3 853 238 B1**
(45) Date of publication and mention of the grant of the patent: **04.11.2026**
(21) Application number: 19790789.2
(22) Date of filing: 19.09.2019
(51) Int. Cl.: A61K 8/60, A61K 9/06

(54) **USE OF AN AMINO ACID DERIVATIVE OF GLUCOSAMINE FOR THE TOPICAL NON-THERAPEUTIC TREATMENT OF SKIN AGING**
ERWENDUNG EINES AMINOSÄUREDERIVATS VON GLUCOSAMIN FÜR DIE TOPISCHE NICHTTHERAPEUTISCHE BEHANDLUNG DER HAUTALTERUNG
UTILISATION D'UN DÉRIVÉ D'ACIDE AMINÉ DE LA GLUCOSAMINE POUR LE TRAITEMENT TOPIQUE NON THÉRAPEUTIQUE DU VIEILLISSEMENT CUTANÉ

(30) Priority: 20.09.2018 IT 201800008758
(43) Date of publication of application: 28.07.2021
(73) Proprietor: Cartilago S.r.l., 73024 Maglie (LC) (IT)
(72) Inventor: SURACE, Filippo, 73024 Maglie (LE) (IT); SCANDURRA, Roberto, 00154 Roma (RM) (IT)
(74) Representative: Sagittarius IP
(86) International application number: PCT/IB2019/057896
(87) International publication number: WO 2020/058889

(56) References cited:
- IT-A1- RM20 090 369
- D. STOPPOLONI ET AL: "Effect of glucosamine and its peptidyl-derivative on the production of extracellular matrix components by human primary chondrocytes", OSTEOARTHRITIS AND CARTILAGE, vol. 23, no. 1, 1 January 2015 (2015-01-01), AMSTERDAM, NL, pages 103 - 113, XP055346705, ISSN: 1063-4584, DOI: 10.1016/j.joca.2014.09.005
- ANNA SCOTTO D'ABUSCO ET AL: "Effects of intra-articular administration of glucosamine and a peptidyl-glucosamine derivative in a rabbit model of experimental osteoarthritis: a pilot study", RHEUMATOLOGY INTERNATIONAL ; CLINICAL AND EXPERIMENTAL INVESTIGATIONS, SPRINGER, BERLIN, DE, vol. 28, no. 5, 9 October 2007 (2007-10-09), pages 437 - 443, XP019587694, ISSN: 1437-160X
- F. VERONESI ET AL: "Chondroprotective activity of N-acetyl phenylalanine glucosamine derivative on knee joint structure and inflammation in a murine model of osteoarthritis", OSTEOARTHRITIS AND CARTILAGE., 1 November 2016 (2016-11-01), GB, XP055347292, ISSN: 1063-4584, DOI: 10.1016/j.joca.2016.10.021
- GIORDANO C ET AL: "Synthesis and properties of d-glucosamine N-peptidyl derivatives as substrate analog inhibitors of papain and cathepsin B", EUROPEAN JOURNAL OF MEDICINAL CHEMISTRY, EDITIONS SCIENTIFIQUE ELSEVIER, PARIS, FR, vol. 26, no. 8, 1 November 1991 (1991-11-01), pages 753 - 762, XP023870484, ISSN: 0223-5234, [retrieved on 19911101], DOI: 10.1016/0223-5234(91)90001-4
- DOHERTY DAVID G ET AL: "Amino acid derivatives of D-glucosamine", JOURNAL OF THE AMERICAN CHEMICAL SOCIETY, AMERICAN CHEMICAL SOCIETY, vol. 75, no. 14, 1 July 1953 (1953-07-01), pages 3466 - 3468, XP009129110, ISSN: 0002-7863, DOI: 10.1021/JA01110A048

## Description

### Background of the invention

As is known, the human body and its component organs are supported and held together by tissues traditionally referred to as connective, a term that indicates their structural and supporting role. However, the function of the connective tissues goes far beyond that of support, acting as a link for the other tissues, participating in the defense mechanisms of the organism and ensuring the nourishment to tissues that are not vascularized.

All the different types of connective tissues consist of two essential components: a cell population and an extracellular matrix (ECM), the latter consisting of an amorphous component and a fibrous component and in which the relative proportions of each of these two components vary depending on the type of connective tissue considered.

The extracellular matrix (ECM) is an organized network of extracellular materials present in the vicinity of the plasma membrane, which can play a key role in determining the shape and activities of a cell. Its components are subjected to a continuous remodeling necessary for the proper functioning of different physiological processes, with a rhythm that slows down with age. Therefore, an inadequate remodeling is at the base of the onset of different diseases that among other things concern both the locomotor apparatus and the skin (Naba et al, 2015).

D'Abusco et al. (2007) discloses the effects of glucosamine (GlcN) and it's N-acetyl-phenylalanine derivative (NAPA) in a Vitamin A model of osteoarthritis in rabbits.

Veronesi et al. (2016) discloses the effects of the N-acetyl-phenylalanine derivative of glucosamine (NAPA) on counteracting osteoarthritis progression in an *in vivo* mouse model.

ITRM20090369A1 relates to the N-acetyl-phenylalanine derivative of glucosamine having characteristics of a selective inhibitor of IKKα.

Giordano et al. (1991) discloses a range of N-peptidyl derivatives of glucosamine.

Doherty et al. (1953) discloses a series of derivatives of D-glucosamine, substituted on the nitrogen atom by acyl amino acid residues.

Stoppoloni et al. (2015) discloses the effects of glucosamine (GlcN) and its peptidyl-derivative, 2-(N-Acetyl)-1-phenylalanylamido-2-deoxy-β-d-glucose (NAPA), on extracellular matrix (ECM) synthesis in human primary chondrocytes (HPCs).

### Brief description of the invention

The compounds disclosed in the present patent application have been discovered to exert surprising and unexpected biological effects. In particular, the compounds referred to or the pharmaceutical compositions comprising them have shown an ability to appropriately stimulate the components of the (ECM) in the connective tissue causing it to reacquire a structure completely similar to the pre-existing healthy structure, or in the case of cartilage without the degenerative signs, for example of osteoarthrosic nature.

Said inventive compounds generally consist of amino acid derivatives of glucosamine and specifically represented by an N-acetyl-phenylalanine derivative of glucosamine, on whose compound the majority of the experimental tests which follow are concentrated.

What just stated derives in general from an in-depth study of the inventors performed with *in vitro* and *in vivo* methods, able to validate the mechanism of action and the biological activity of the innovative amino acid derivatives of glucosamine.

The above class of compounds and more specifically the N-acetyl-phenylalanine derivative of glucosamine, has been studied in *in vivo* models of OA both in rabbit and in mice, finding that the intra-articular administration of said compound in the osteoarthritic knees of rabbits or mice stimulated the synthesis of new ECM, making the cartilage regain a structure very similar to the healthy one, free of osteoarthritis and thus suggesting that this class of compounds has an important regenerative effect.

To clarify the molecular mechanism, glucosamine-derived N-acetyl-phenylalanine has been studied *in vitro,* using primary cultured chondrocytes. The chondrocytes were stimulated with IL-1 in order to study the inflammatory pathway, controlled by the Activator Protein (AP)-1. Specifically, said N-acetyl-phenylalanine glucosamine derivative was unexpectedly effective in first inhibiting the phosphorylation of two kinases, JNK and p38, and consequently influencing the activation of c-jun, one of the components of AP-1. The efficacy of interfering with the inflammatory pathway controlled by the transcription factor Nuclear Factor-κB (NF-κB) was then analyzed, which in turn is controlled by the IKK complex consisting of two IKKα and IKKβ kinases. Said N-acetyl-phenylalanine derivative of glucosamine inhibited the translocation in the nucleus of the IKKα kinase, which exerts its activity above all in that cellular district, selectively inhibiting the kinase activity of IKKα but not IKKβ.

Finally, in order to understand which intracellular pathways are activated by said N-acetyl-phenylalanine derived from glucosamine to stimulate ECM synthesis, further *in vitro* studies in human primary chondrocytes were conducted. In order to simulate physiological conditions, the culture of human chondrocytes was carried out in three dimensions, cultivating the cells in the form of spheroids. The results unexpectedly showed that the aforementioned inventive compound stimulated the synthesis of type II collagen, characteristic of cartilage tissue. In addition, through these experiments it was found that if the cells are treated simultaneously with glucosamine and said N-acetyl-phenylalanine derived from glucosamine, a synergistic effect is obtained, with an even greater production of type II collagen, inducing the expression of a growth factor, IGF-1 (Insulin Growth Factor), and a transcription factor SOX-9 (Sry-related HMG bOX-containing genes), which control the synthesis of ECM, thus determining this interesting anabolic effect exercised by the association of the two molecules.

Another important and unexpected discovery is that the aforementioned amino acid derivatives of glucosamine and more specifically the N-acetyl-phenylalanine derivative of glucosamine is able to stimulate the expression of the enzyme synthetase of hyaluronic acid (HAS-2) and consequently the production of hyaluronic acid (HA).

Another promising discovery for the purposes of carrying out the present invention is that the aforementioned class of inventive compounds has proved to be particularly effective also on primary human fibroblasts, which when grown in three dimensions, like spheroids, have produced a rather high amount of type I collagen with respect to spheroids grown in the absence of said innovative compound. Furthermore, the N-acetyl-phenylalanine derivative of glucosamine has also been added to the culture medium of fibroblasts stimulated with UVB, to such an extent as to simulate the irradiation of solar UVB, finding that the molecule was able to counteract the inflammatory pathways stimulated by UVB and also to restore the synthesis of type I collagen, which is instead blocked by UVB treatment. This discovery promptly aroused in the inventors an interest in the ability of this class of products to intervene in the remodeling of the cutaneous ECM, in which the relative alteration of turnover leads to atrophy of the skin, with consequent aging of the skin and in some cases even to onset of skin cancer processes.

Therefore, starting from the promising results described both in the aforementioned *in vivo* and *in vitro* studies, the inventors of the following invention have provided use of a pharmaceutical composition suitable for the topical administration route comprising an amino acid derivative of glucosamine having the following structural Formula 2: 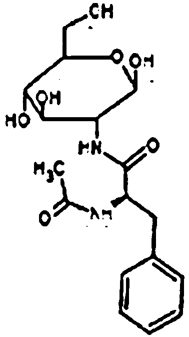 or the pharmaceutically acceptable salt, solvate, hydrate or stereoisomer thereof, in a base cream, characterized in that said amino acid derivative of glucosamine is included in said pharmaceutical composition at a concentration of between 0.1% by weight and 2% by weight in the base cream, for the non-therapeutic treatment of skin aging.

Other features of the present invention are described in the following detailed description of one or more specific embodiments thereof, protected by the various dependent claims.

### Detailed description of the invention

It should be noted that hereinafter, only some of the possible embodiments of the present invention will be illustrated, by way of non-limiting example, it being possible to describe many others thereof on the basis of the particular technical solutions identified.

The present patent application relates to use of a pharmaceutical composition suitable for the topical administration route comprising an amino acid derivative of glucosamine having the following structural Formula 2: 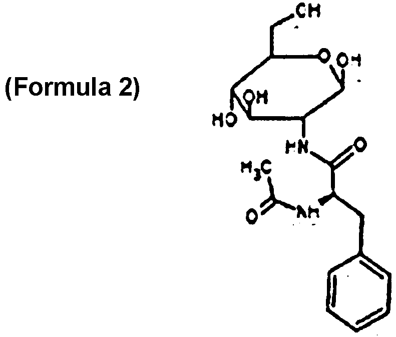 or the pharmaceutically acceptable salt, solvate, hydrate or stereoisomer thereof, in a base cream, characterized in that said amino acid derivative of glucosamine is included in said pharmaceutical composition at a concentration of between 0.1% by weight and 2% by weight in the base cream, for the non-therapeutic treatment of skin aging.

A further object of the invention relates to the aforementioned ability of this class of products to restore the synthesis of type I and type II collagen due to the activity of fibroblasts, intervening in the remodeling of the cutaneous ECM and therefore to be used appropriately in the non-therapeutic treatment of skin aging by counteracting skin atrophy.

The inventive compound is an N-acetyl-phenylalanine derived from glucosamine having the following structural Formula 2:

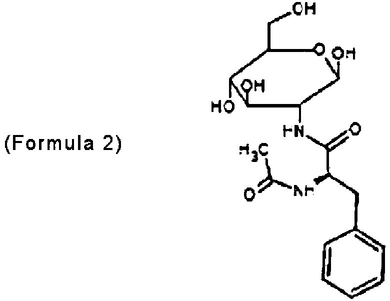

The pharmaceutical composition which contains the compound having structural Formula 2 may contain it alone or in association with one or more active ingredients, together with one or more excipients or carriers suitable for the route of administration chosen and prepared according to the well-known standards of good manufacturing technology and pharmaceutical legislation.

The present invention has the object of providing a pharmaceutical composition suitable for the topical administration route by making a cream consisting of a compound having structural Formula 2 or of a pharmaceutically acceptable salt, solvate, hydrate or stereoisomer thereof at a concentration comprised between 0.1% by weight and 2% by weight and more preferably equal to 1% by weight in the base cream.

## Claims

1. Use of a pharmaceutical composition suitable for the topical administration route comprising an amino acid derivative of glucosamine having the following structural Formula 2: 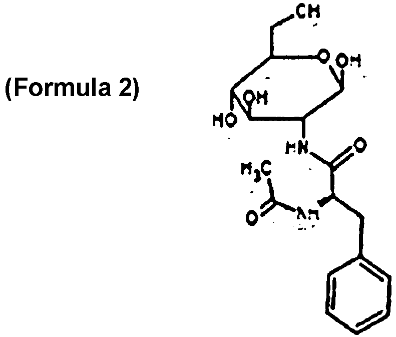 or the pharmaceutically acceptable salt, solvate, hydrate or stereoisomer thereof, in a base cream, **characterized in that** said amino acid derivative of glucosamine is included in said pharmaceutical composition at a concentration of between 0.1% by weight and 2% by weight in the base cream, for the non-therapeutic treatment of skin aging.

2. The use according to claim 1, **characterized in that** said amino acid derivative of glucosamine is included in said pharmaceutical composition at a concentration of 1% by weight in the base cream.

3. The use according to claim 1 or claim 2, wherein the pharmaceutical composition is administered by topical administration.

## Patentansprüche

1. Verwendung einer pharmazeutischen Zusammensetzung, die für den topischen Verabreichungsweg geeignet ist, umfassend ein Aminosäurederivat von Glucosamin, das die folgende Strukturformel 2 aufweist: 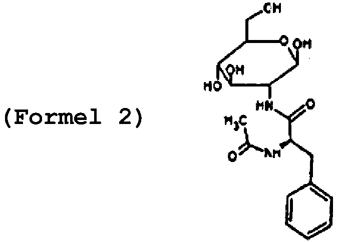 oder das pharmazeutisch verträgliche Salz, Solvat, Hydrat oder Stereoisomer davon, in einer Basiscreme, **dadurch gekennzeichnet, dass** das Aminosäurederivat von Glucosamin in der pharmazeutischen Zusammensetzung in einer Konzentration von zwischen 0,1 Gew.-% und 2 Gew.-% in der Basiscreme enthalten ist, für die nichttherapeutische Behandlung von Hautalterung.

2. Verwendung nach Anspruch 1, **dadurch gekennzeichnet, dass** das Aminosäurederivat von Glucosamin in der pharmazeutischen Zusammensetzung in einer Konzentration von 1 Gew.-% in der Basiscreme enthalten ist.

3. Verwendung nach Anspruch 1 oder Anspruch 2, wobei die pharmazeutische Zusammensetzung durch topische Verabreichung verabreicht wird.

## Revendications

1. Utilisation d'une composition pharmaceutique adaptée à la voie d'administration topique comprenant un dérivé d'acide aminé de la glucosamine ayant la formule structurale 2 suivante : 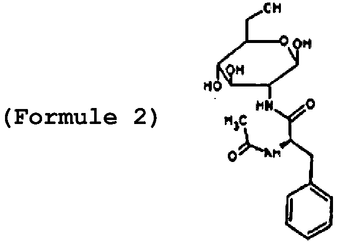 ou le sel, solvate, hydrate ou stéréoisomère pharmaceutiquement acceptable de celui-ci, dans une crème de base, **caractérisée en ce que** ledit dérivé d'acide aminé de la glucosamine est inclus dans ladite composition pharmaceutique à une concentration comprise entre 0,1 % en poids et 2 % en poids dans la crème de base, pour le traitement non thérapeutique du vieillissement cutané.

2. Utilisation selon la revendication 1, **caractérisée en ce que** ledit dérivé d'acide aminé de la glucosamine est inclus dans ladite composition pharmaceutique à une concentration de 1 % en poids dans la crème de base.

3. Utilisation selon la revendication 1 ou la revendication 2, dans laquelle la composition pharmaceutique est administrée par administration topique.
